# EUROPEAN PATENT APPLICATION

(11) **EP 4 328 315 A1**
(43) Date of publication of application: **28.02.2024**
(21) Application number: 21725561.1
(22) Date of filing: 19.04.2021
(51) Int. Cl.: C12P 5/02, C05F 17/50, B09B 3/40

(54) **PRODUCTION PROCESS FOR PRODUCING BIOGAS BY MEANS OF ANAEROBIC CO-DIGESTION**

(71) Applicant: ECONWARD TECH, S.L., 28014 Madrid (ES)
(72) Inventor: APARICIO GAYA, Julio César, 28014 Madrid (ES); SALGUERO CARVAJAL, Alberto, 28014 Madrid (ES); SOLER, Julián Alberto, 28014 Madrid (ES); MENA SANZ, Javier, 13500 Puertollano (Ciudad Real) (ES); GARCÍA CANO, Rubén, 28014 Madrid (ES)
(74) Representative: Ungria López, Javier
(86) International application number: PCT/ES2021/070256
(87) International publication number: WO 2022/223852

(57) **Abstract**

The invention relates to a production process for producing biogas by means of anaerobic co-digestion which comprises: (a) preparing hydrolyzed biomass from organic solid waste by means of a thermal hydrolysis treatment of waste at between 1.5 and 4.5 bar and between 120 and 160 °C, generating raw biomass from which foreign matter is separated, giving rise to clean hydrolyzed biomass with at least 90 % organic matter, a volatile solids to total solids ratio of at least 0.6 and at least 5 % total solids; (b) mixing the clean hydrolyzed biomass with sludge from a wastewater treatment plant (WWTP), generating a mixture with a concentration of solids of less than 30 % which is conditioned to a concentration of less than 20 %; and (c) a step of wet anaerobic digestion in a digester at between 25 °C and 40 °C or between 50 °C and 60 °C and during an HRT of between 12 and 30 days, generating biogas and a digestate.

## Description

### SECTOR OF THE ART

The object of the present invention pertains to the technical field of environmental technology and, particularly, relates to a new production process for producing biogas by means of anaerobic co-digestion of organic waste: sludge originating from urban wastewater (hereinafter, UWW) and organic solid waste (hereinafter, OSVW, understanding as such both the organic fraction of municipal solid waste (hereinafter, MSW) and assimilable waste, preferably organic waste originating from the selective collection of MSW, as well as organic waste from agriculture, horticulture, aquiculture, forestry, hunting, fishing or the preparation and production of food and industrial organic waste, among others.

### BACKGROUND OF THE INVENTION

The present invention arises from the need for improvement of current municipal waste management processes. Particularly, it relates to a new process for optimizing the production of biogas by means of the treatment of two types of organic waste which, to date, are managed independently, namely: OSW and sludge originating from UWW.

The treatment of UWW in treatment plants comprises, mainly, two process lines: on one hand, the water is treated to enable dumping it into a watercourse and/or reusing it to clean streets and/or irrigate gardens and, on the other hand, the solid fraction or sludge that is separated or generated during wastewater treatment is managed. In this case, the sands or bulky waste are removed and destined for a dump and, on the other hand, the sludge generated during biological treatment and/or removed during steps of settling is mostly subjected to a wet anaerobic digestion process.

In the case of municipal solid waste and assimilable waste, the usual treatment consists of separating the different fractions present therein (plastics, metals and organic fraction, mostly) to subsequently treat and recover each fraction independently. In the case of the organic fraction, the main forms of recovery are: composting or aerobic stabilization, anaerobic digestion or thermal recovery (incineration, gasification, pyrolysis, etc.).

In the case of anaerobic digestion, there are two main trends in waste management:
- the dry process, when the concentration of solids is greater than 20 % by weight, and
- the wet process, when the concentration of solids is less than 20 % by weight (generally, around 10 or 12 %).

A number of inventions relating to sludge treatment processes by means of anaerobic digestion can be found in patent literature.

Thus, for example, patent US3338826 relates to a process for wastewater treatment by means of anaerobic digestion which comprises subjecting wastewater to a pressure of between 2 and 4 atmospheres to accelerate decomposition.

In turn, patent EP0737651 relates to a method for wastewater treatment which comprises, among other steps, subjecting a surplus sludge to thermal pre-treatment at a temperature of 60 °C or higher, dehydrating the surplus sludge and mixing the dehydrated sludge with the digested sludge resulting from a methane fermentation process, such that the obtained mixture is used as a substrate of a new methane fermentation process.

Although there have been many solutions proposed for optimizing the process for treating wastewater sludge generated in wastewater treatment plants (WWTP), to date there has been no solution found which is based on conditioning, by means of a thermal hydrolysis process, another type of organic waste, preferably solid waste originating from the selective collection of the organic fraction of MSW, said waste being used, once it has been treated, to improve the efficiency of the process for the anaerobic digestion of sludge generated or separated in wastewater sludge treatment plants. Unlike the solution object of the present invention, processes known in the state of the art based on joint treatment by means of the co-digestion of organic waste and sludge from a treatment plant are based on the use of raw organic waste, in other words, waste not subjected to any prior treatment, beyond steps of eliminating foreign matter.

As a result of the thermal hydrolysis treatment of organic solid waste, multiple advantages are achieved, as described in detail below. Among these, the surprising results that are achieved with respect to the generation and quality of the obtained biogas should be pointed out, contributing to the self-supply of energy in treatment facilities and even the generation of surplus.

### DESCRIPTION OF THE INVENTION

In this manner, the object of the invention is a production process for producing biogas by means of anaerobic co-digestion, characterized in that it comprises:
(a) a first step of preparing hydrolyzed biomass from organic solid waste. This first step in turn comprises:
   i. a first sub-step comprising a thermal hydrolysis treatment of organic solid waste. This thermal hydrolysis treatment can preferably be carried out by subjecting waste to a pressure comprised between 1.5 and 4.5 bar and a temperature of between 120 and 160 °C, for a time that can range between 10 and 75 minutes. Nevertheless, the pressure and time may vary to optimize the effects of thermal hydrolysis without generating inhibitory compounds which may affect the following steps of the process.
      The following benefits are obtained by means of this thermal hydrolysis treatment:
      - waste sanitation;
      - partial degradation of the organic fraction, without affecting the foreign matter (understanding as such matter the waste fractions or components that are not susceptible to being recovered in an anaerobic digestion process). This results in:
         - homogenization of the organic fraction,
         - an increase in the foreign matter separation efficiency, whereby obtaining greater utilization of the organic fraction, minimizing loss in a subsequent cleaning process;
      - thermal and biological stabilization of the organic matter;
      - transformation of complex compounds into soluble and more readily degradable molecules.
      The result of this step of thermal hydrolysis is a product which, for purposes of this patent, will be referred to as "raw biomass". Although the characteristics of the "raw biomass" may largely vary depending on the source of the starting waste, in a particular embodiment, said "raw biomass" is characterized in that it comprises: between 70 and 75 % by weight of biodegradable organic matter, between 10 and 15 % by weight of lightweight foreign matter (understanding as such materials which, when the organic matter is solubilized, tend to float as they have lower densities than the suspension of the organic matter, such as, for example, textiles, plastics, plastic-cardboard containers, wood pruning waste, wood or corks, etc.) and between 10 and 15 % by weight of heavy foreign matter (understanding as such materials which, when the organic matter is solubilized, tend to precipitate as they have higher densities than the suspension of the organic matter, such as, for example, glass, plate glass, sand, stones, bones, etc.);
   ii. a second sub-step is then carried out for the post-treatment of the raw biomass, separating foreign matter. In particular, the separation of lightweight foreign matter and heavy foreign matter is carried out.
      The removal of foreign matter can be carried out by means of any method known in the state of the art for carrying out said separation process, for example by means of using trommel-type separators, vibrating tables or depackers for the elimination of lightweight foreign matter, and settling tanks, hydrocyclones, hydroclassifiers or sand traps, among others, for the elimination of heavy foreign matter.
      As a result of this sub-step, a hydrolyzed biomass is obtained which, for purposes of this patent, will be referred to as "substrate" or "clean hydrolyzed biomass", which may either be stored or continue in the process. This substrate is characterized in that it comprises a percentage of organic matter of at least 90 %, and more preferably of at least 98 % by weight. Preferably, the volatile solids to total solids weight ratio in said product will be at least 0.6, and more preferably at least 0.8, and its total solids content of at least 5 % by weight;
(b) a second step of mixing the clean hydrolyzed biomass obtained in the previous step with the sludge from a wastewater treatment plant (WWTP), giving rise to a mixture with a concentration of solids of less than 30 % by weight and generally between 5 and 15 % by weight. Preferably, the amount of clean hydrolyzed biomass in the mixture may range between 5 and 65 % by weight, while the amount of sludge may range between 35 and 95 % by weight. Nevertheless, the final amount of each component in the mixture is not limiting, where it may vary depending on seasonality or other circumstances.
   In this manner, the claimed process allows a constant production of biogas to be maintained over the year, regardless of the variation that may exist in the production of sludge (for example, in urban areas with changes in population over the year). In these cases, a larger amount of hydrolyzed biomass may be used to compensate for periods in which less sludge is produced, thus maintaining the production of biogas.
   The obtained mixture is then subjected to a conditioning process to adjust the amount of total solids to a percentage of less than 20 % by weight and preferably between 5 and 15 % by weight, said amount being the amount required for carrying out the following step of the process;
(c) a third step of wet anaerobic digestion of the mixture obtained in the previous step in at least one digester, giving rise to biogas and a digestate.
   Preferably, anaerobic digestion will be carried out under mesophilic conditions (at a temperature of between 25 °C and 40 °C and more preferably between 35 °C and 38 °C) and during a hydraulic retention time (HRT) of between 12 and 30 days. In other particular embodiments of the invention, the anaerobic digestion may be carried out under thermophilic conditions (at a temperature of between 50 °C and 60 °C).
   In turn, the pH of the anaerobic digestion process will preferably range between 7 and 8.5 and the organic loading rate between 1.5 and 5 kg VS /m³ day.

In a particular embodiment of the invention in which the organic solid waste is the organic fraction of municipal solid waste and assimilable waste, the process may comprise a prior step of preparing waste by means of separating bulky foreign matter (understanding as such those large-volume fractions or components readily separable by size, the size thereof generally being greater than 80 mm) and/or metals present therein. In particular, the separation of the bulky waste can be carried out by means of at least one trommel-type rotating separator with a mesh size preferably comprised between 60 and 120 mm. In turn, in the case of the presence of ferrous metals, said metals may be separated by means of at least one magnetic separator.

Likewise, the process may particularly comprise an additional step of utilizing the biogas obtained, preferably by means of cogeneration (producing both heat and electricity), in boilers for the generation of heat, or being subjected to a purification (upgrading) process for obtaining biomethane.

Additionally, the process may comprise an additional step of utilizing the digestate, preferably by means of agricultural use as a biofertilizer.

For purposes of this patent, organic solid waste is understood as both the organic fraction of the MSW and assimilable waste (and preferably the organic waste originating from the selective collection of MSW), as well as organic waste from agriculture, horticulture, aquiculture, forestry, hunting, fishing or the preparation and production of food, industrial organic waste, etc.

In turn, MSW is understood as any household waste, in other words, any substance or object which has been discarded in homes as a consequence of household activities. MSW assimilable waste is considered waste similar to the aforementioned generated in shops, industries and institutions, for example market waste, street cleaning waste, sewage cleaning waste, etc.

Particularly, organic solid waste is characterized in that it is waste susceptible to being biologically degraded and can comprise, without limitation: fruit and vegetable waste, meat and fish waste, egg shells, shells from shellfish and dried fruits and nuts or other food waste, waste from infusions and coffee grounds, used napkins, dirty paper towels and dirty paper and cardboard from oil or food waste, small garden waste (plants, fallen leaves or bouquets of flowers), etc.

Furthermore, for purposes of this patent, sludge from a wastewater treatment plant (WWTP) is understood as the mixture of water and solids obtained in a plant that treats household or municipal wastewater or the mixture thereof with industrial wastewater and/or rainfall runoff water. The composition will therefore be variable, depending on the composition of the initial wastewater, the origin thereof and/or the type of treatment the wastewater is to be subjected. In general, the percentage of water in sludge will be greater than 95 % by weight.

There are many advantages derived from the process object of the invention based on the use of organic solid waste (once it is subjected to a thermal hydrolysis process) as a substrate in the anaerobic digestion process for digesting the sludge from a WWTP. Particularly, the makeup of hydrolyzed biomass as the substrate for anaerobic digestion results in:
(1) utilizing a biowaste (the organic fraction of the MSW and assimilable waste or the organic waste of different origins, as previously described, once hydrolyzed) in existing facilities (in particular, the facilities of a WWTP plant), which are currently energy deficient;
(2) integrating waste management, which is conventionally managed separately;
(3) utilizing the existing facilities of the WWTP, avoiding new investments, although new plants integrating the management of different waste in one and the same facility could also be built;
(4) increasing not only the concentration of total solids but also the concentration of volatile solids by volume of digester and, therefore, the organic loading rate (OLR). In particular embodiments of the invention, an increase in the OLR of between 13 and 108 % has been achieved. In addition to increasing the organic loading rate of the digester, a constant organic loading rate is maintained, regardless of seasonality;
(5) surprisingly increasing the production of biogas as a result of the synergistic effect achieved by means of the claimed mixture of waste. In particular embodiments, an increase in the production of biogas of between 15 and 124 % has been achieved with respect to the production achieved in current WWTPs with sludge/biomass ratios of between 93/7 and 80/20. As a consequence of this important increase in the production of biogas, not only is a higher production of renewable energy achieved, but also a contribution is made to the self-supply of energy of the treatment facility, even generating a surplus;
(6) improving the quality of the biogas, increasing the percentage of CH₄. In a preferred embodiment of the invention, the biogas obtained by means of the claimed process can comprise between 64.6 and 67.4 % of CH₄ and between 35.4 and 32.6 % of CO₂. The fact that the obtained biogas has a better quality than the one obtained with sludge treatment today in WWTPs, combined with the increase in production, is an important advantage in many ways. Particularly, it allows a higher percentage of electricity to be obtained in combined cycles, improving the percentage of self-supply. Additionally, in the case of achieving self-supply, the surplus biogas can be used for other purposes, for example for obtaining biomethane by means of a purification (*upgrading*) process, which can be sold, obtaining extra income for the water treatment plant;
(7) increasing between 1 and 10 % the lower heating value (LHV) of the fuel due to the increase in the concentration of methane in the biogas;
(8) reducing needs for deodorizing and enriching (*upgrading*), which represents a savings in reagents and lower operating and maintenance costs. Likewise, the fact that it has a higher quality allows elongating the service life of cogeneration engines in which it is utilized;
(9) increasing process stability given that:
   - the organic loading rate of the digester is increased and the hydraulic retention time (HRT) is reduced between 5 and 60 %;
   - the use of chemical reagents for process control is minimized; and
   - degradability of the organic matter of the digester is increased, increasing the reduction of total solids and volatile solids between 6 % and 27 % with respect to current processes.

Likewise, the fact that the process is based on the use of a hydrolyzed biomass allows a series of additional advantages to be obtained over processes that are based on the use of non-hydrolyzed organic waste, as described below:
- first, higher biodegradability of the organic waste is achieved, which results in a higher production of biogas;
- moreover, the separation of foreign matter in the process is more efficient. Particularly, during the usual process of cleaning foreign matter in the MSW, between 30 and 40 % by weight of organic matter is lost, which hinders being able to obtain a quality substrate, such as the one achieved as a result of the claimed process. In this manner, as a result of the hydrolysis process object of the invention, the loss of organic matter in the process of cleaning foreign matter is less than 5 % by weight, which represents a higher utilization of the organic matter present in the starting waste;
- in turn, as a result of waste sanitation, the extra addition of pathogens which may affect digester operation is prevented;
- finally, as a result of thermal and biological stabilization, the emission of odors associated with decomposition of organic matter is minimized.

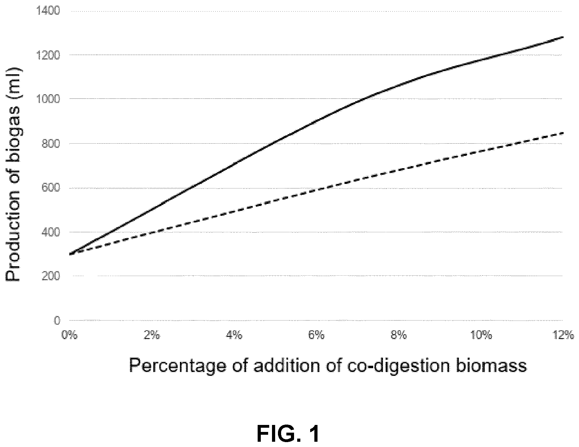

### BRIEF DESCRIPTION OF FIGURE 1

To complement the present description, the following figure is attached as an integral part thereof:
**Fig. 1** shows a comparative graph of the theoretical production of biogas versus the production of biogas obtained by means of the process object of the invention.

### PREFERRED EMBODIMENT OF THE INVENTION

A particular embodiment of the invention is described below for the purpose of demonstrating the advantages of the claimed process described above. Particularly, said particular embodiment was carried out based on organic solid waste originating from the selective collection of MSW in the city of Madrid, the composition of which is shown in the following table:

**Table 1. Composition of the OSW used in the process**

| **Fraction** | **Percentage (% by weight, wet base)** |
|---|---|
| Food waste* | 63.7 |
| Garden and pruning waste | 8.7 |
| LWC (Lightweight Containers) | 8.0 |
| Plastic | 0.7 |
| Paper/cardboard | 3.8 |
| Celluloses | 1.4 |
| Textiles | 2.6 |
| Glass | 3.0 |
| Other waste | 8.1 |

| | |
|---|---|
| * Fruit and vegetable waste, meat and fish waste, egg shells, shells from shellfish and dried fruits and nuts or other food waste, infusions, coffee grounds, etc. | |

Said waste was subjected to the following steps:
- a prior step of preparing waste by means of separating bulky foreign matter carried out in a trommel with a mesh size of 80 mm;
- a step of preparing hydrolyzed biomass from the organic solid waste obtained in the previous step. In turn, said step comprised:
   - a first sub-step of thermal hydrolysis treatment at a pressure of 4 bar and at a temperature of 150 °C for a time of 20 minutes;
   - a second sub-step of eliminating lightweight foreign matter by means of using a depacker, as well as eliminating heavy foreign matter by means of a sand trap.

At the end of this step, a "substrate" or "clean hydrolyzed biomass" with an organic content of 98 % by weight was obtained. Said substrate was used in the co-digestion tests described below.

Particularly, to carry out said tests, two blanks characterized in that they comprised only anaerobic sludge (ANS) and four samples with a variable aerobic sludge (AES) and clean hydrolyzed biomass ratio were prepared. Particularly, the prepared samples were the following:

**Table 2. Description of the samples, % by weight**

| **Mixture** | **WWTP sludge (AES)** | **Hydrolyzed biomass** |
|---|---|---|
| Sample 1 (AES) | 100.0 % | 0 % |
| Sample 2 (93:7) | 93.0 % | 7.0 % |
| Sample 3 (88:12) | 88.0 % | 12.0 % |
| Sample 4 (80:20) | 80.0 % | 20.0 % |

**Table 3. Mass (g) of each of the samples**

| **Mixture** | **ANS mass** | **AES mass** | **Mass Sample 2 (93:7)** | **Mass Sample 3 (88:12)** | **Mass Sample 4 (80:20)** |
|---|---|---|---|---|---|
| Blank 1 | 338.4 | 0 | 0 | 0 | 0 |
| Blank 2 | 338.4 | 0 | 0 | 0 | 0 |
| Sample 1 | 338.4 | 161.6 | 0 | 0 | 0 |
| Sample 2 (93:7) | 338.4 | 0 | 150.2 | 0 | 0 |
| Sample 3 (88:12) | 338.4 | 0 | 0 | 143 | 0 |
| Sample 4 (80:20) | 338.4 | 0 | 0 | 0 | 132.8 |

The main properties of each of the samples are summarized in the following table, wherein the methods of measuring the different parameters were the following:
- Total solids (TS): APHA 2540 B
- Volatile solids (VS): APHA 2540 E
- COD: APHA 5220 D
- COD sol.: APHA 5220 D
- VFAs: APHA 2310 B

**Table 4 Main characteristics of the samples of the invention**

| **Parameter** | **Unit** | **AES sludge** | **Hydrolyzed biomass** | **ANS sludge** |
|---|---|---|---|---|
| Total solids (TS) | % weight | 5.94 | 9.35 | 4.21 |
| Volatile solids (VS) | % weight | 2.89 | 6.02 | 1.38 |
| COD | ppm | 69750 | 86375 | 18650 |
| COD sol | ppm | 5800 | 24175 | 705 |
| VFAs | mg/ l CaCO₃ | 5237 | 13251 | 141 |

Water was added to each of the samples until achieving a total weight of 500 g. Each sample was prepared in triplicate.

The prepared samples were subjected to an anaerobic digestion process at a temperature of 35 °C. No nutrients or buffers were added.

Next, an analysis of the obtained results was carried out, and these results are shown in the following tables:

**Table 5. Result of TS and VS elimination**

| **Sample** | **Start % TS** | **End % TS** | **Start %VS** | **End %VS** |
|---|---|---|---|---|
| Blank | 0.6 | 0.54 | 0.42 | 0.28 |
| Sample 1 | 1.26 | 0.92 | 0.8 | 0.5 |
| Sample 2 (93:7) | 0.94 | 0.81 | 0.81 | 0.41 |
| Sample 3 (88:12) | 0.96 | 0.88 | 0.79 | 0.43 |
| Sample 4 (80:20) | 1.18 | 1.02 | 0.92 | 0.57 |

It has therefore been demonstrated that the percentage of VS/TS increases considerably when using hydrolyzed biomass originating from the organic fraction of the MSW as a substrate of the digester. Particularly, the percentage of VS/TS was 69 % in the blanks (AES), 75 % in sample 1 (ANS) and 80 to 85 % in samples 2 to 4, which confirms the increase in biodegradability of the mixtures when feeding the digester with hydrolyzed biomass.

**Table 6. Result of BOD and VFA elimination**

| **Sample** | **Start BODt (mg/l)** | **End BODt (mg/l)** | **Start Total VFAs (mg/l)** | **End Total VFAs (mg/l)** |
|---|---|---|---|---|
| Blank | 4350 | 3785 | 259 | 26.5 |
| Sample 1 | 10367 | 6178 | 2221.7 | 15.7 |
| Sample 2 (93:7) | 10620 | 4690 | 2842.3 | 34.4 |
| Sample 3 (88:12) | 10533 | 5290 | 2232.2 | 18.6 |
| Sample 4 (80:20) | 13100 | 7332 | 2564.8 | 31.3 |

The preceding results demonstrate that even when the amount of VS is the same, the COD increases with the presence of hydrolyzed biomass, as occurs with the VFAs, which confirms higher biodegradability of the samples.

**Table 7. Specific CH₄ production results by eliminated BOD**

| **Mixture** | **Production (I CH₄/kg eliminated BOD)** | **Increase*** | |
|---|---|---|---|
| | | **I CH₄/kg eliminated BOD*** | **Increase** |
| Sample 1 (control) | 48.5 | 0 | 0% |
| Sample 2 (93:7) | 112.6 | 64.1 | 132.2 % |
| Sample 3 (88:12) | 108.6 | 60.1 | 123.9% |
| Sample 4 (80:20) | 213.8 | 165.3 | 340.8 % |

| | | | |
|---|---|---|---|
| * Represents the increase in the production of CH₄ of each of the samples with respect to sample 1 | | | |

The results demonstrate the synergy achieved as a result of the mixture of the hydrolyzed biomass obtained by means of a process of hydrolysis of the organic fraction of the MSW, with the sludge from a WWTP. Particularly, the process object of the invention achieves a non-linear production in the generation of biogas, achieving with 20 % hydrolyzed biomass fed into the digester an increase of 340.8 % in the specific production of methane per kilogram of COD eliminated and of 213.8 % per kilogram of COD fed in. It has also been demonstrated that the use of hydrolyzed biomass in the mixture fed into the digester in which the anaerobic digestion process takes place increases the degree of hydrolysis and, therefore, biodegradability.

In addition to the improvement in the production of biogas, an analysis of its methane (CH₄) content was carried out, as shown in the following table:

**Table 8. Percentae (by volume) of methane**

| **Mixture** | **% vol. (CH4)** |
|---|---|
| Sample 1 (control) | 63.3 |
| Sample 2 (93:7) | 64.6 |
| Sample 3 (88:12) | 65.1 |
| Sample 4 (80:20) | 67.4 |

The preceding results demonstrate the improvement in the quality of the obtained biogas by increasing the percentage of hydrolyzed biomass used in the digester.

Lastly, for the purpose of demonstrating the synergy obtained as a result of the process object of the invention, a comparison was carried out between the production of biogas obtained by means of the claimed process with respect to that which would theoretically be obtained by the summation of the biogas produced by the sludge from the treatment plant and the biogas produced by the hydrolyzed biomass. The obtained results are shown in Figure 1. Specifically, as shown in said figure, the real production of biogas (continuous line) is 50 % higher with respect to the maximum theoretical production (discontinuous line) equivalent to the theoretical sum of the production of biogas obtained from both substrates independently (i.e., without mixing them). This is due to the improvements derived from the addition of the hydrolyzed biomass to the sludge from the treatment plant. Particularly, the following aspects are improved:
- the organic carbon makeup, whereby increasing the C/N ratio (generally sludge from the treatment plant is rich in nitrogen and deficient in carbon);
- and the makeup in micronutrients, such as Zn, Co, Fe, K, or P.

The fact that the real production of biogas in co-digestion is 50 % higher than the theoretical maximum demonstrates the synergy that exists when mixing sludge with hydrolyzed biomass.

## Claims

1. A production process for producing biogas by means of anaerobic co-digestion, **characterized in that** it comprises:
(a) a first step of preparing hydrolyzed biomass from organic solid waste in turn comprising:
i. a first sub-step comprising a thermal hydrolysis treatment of the organic solid waste, wherein said thermal hydrolysis treatment is carried out at a pressure comprised between 1.5 and 4.5 bar and a temperature of between 120 and 160 °C, resulting in raw biomass;
ii. a second sub-step is then carried out for the post-treatment of the raw biomass, separating foreign matter and resulting in clean hydrolyzed biomass, **characterized in that** it comprises a percentage of organic matter of at least 90 % by weight, a volatile solids to total solids weight ratio of at least 0.6 and a total solids content of at least 5 % by weight;
(b) a second step of mixing the clean hydrolyzed biomass obtained in the previous step with sludge from a wastewater treatment plant (WWTP), giving rise to a mixture with a concentration of solids of less than 30 % by weight, wherein said mixture is then subjected to a conditioning process until achieving a concentration of solids of less than 20 % by weight;
(c) a third step of wet anaerobic digestion of the mixture obtained in the previous step in at least one digester, giving rise to biogas and a digestate, wherein said anaerobic digestion is carried out under mesophilic conditions of between 25 °C and 40 °C or thermophilic conditions of between 50 °C and 60 °C and during a hydraulic retention time (HRT) of between 12 and 30 days.

2. The process according to claim 1, wherein the mixture of clean hydrolyzed biomass and WWTP sludge comprises between 5 % and 65 % by weight of clean hydrolyzed biomass and between 35 % and 95 % by weight of WWTP sludge.

3. The process according to claim 1 or 2, wherein said process comprises an additional step of utilizing biogas by means of cogeneration, use in boilers for generating heat or use for biomethane production by means of a purification or upgrading process.

4. The process according to any one of the preceding claims, wherein said process comprises an additional step of utilizing the digestate by means of agricultural use as a biofertilizer.

5. The process according to any one of the preceding claims, wherein the organic solid waste is selected from a group consisting of the organic fraction of municipal solid waste and assimilable waste, organic waste originating from the selective collection of municipal solid waste, organic waste from agriculture, horticulture, aquiculture, forestry, hunting, fishing and the preparation and production of food and industrial organic waste, as well as any of the combinations thereof.

6. The process according to claim 5, wherein the organic solid waste is the organic fraction of municipal solid waste and wherein said process comprises a prior step of separating bulky foreign matter, understanding as such matter having a size greater than 80 mm and/or of separating ferrous metals by means of at least one magnetic separator.
